# EUROPÄISCHE PATENTSCHRIFT

(11) **EP 0 593 726 B1**
(45) Veröffentlichungstag und Bekanntmachung des Hinweises auf die Patenterteilung: **06.11.1996**
(21) Anmeldenummer: 93909955.2
(22) Anmeldetag: 11.05.1993
(51) Int. Cl.: B07C 5/34

(54) **KONTINUIERLICH ARBEITENDE INSPEKTIONSMASCHINE FÜR GEFÄSSE**
CONTINUOUS OPERATION INSPECTION MACHINE FOR CONTAINERS
MACHINE D'INSPECTION EN CONTINU DE RECIPIENTS

(30) Priorität: 11.05.1992 DE 4214958
(43) Veröffentlichungstag der Anmeldung: 27.04.1994
(73) Patentinhaber: KRONES AG HERMANN KRONSEDER MASCHINENFABRIK, D-93073 Neutraubling (DE)
(72) Erfinder: GRIESBECK, Karl, D-8400 Regensburg (DE)
(74) Vertreter: Grünecker, Kinkeldey, Stockmair & Schwanhäusser Anwaltssozietät
(86) Internationale Anmeldenummer: EP9301165
(87) Internationale Veröffentlichungsnummer: WO9323180

(56) Entgegenhaltungen:
- EP-A- 0 295 371
- DE-A- 3 611 536
- DE-A- 3 621 976

## Beschreibung

Die Erfindung betrifft eine kontinuierlich arbeitende Inspektionsmaschine für Gefäße gemäß dem Oberbegriff des Anspruchs 1.

Insbesondere in Abfüllanlagen müssen verschmutzte oder beschädigte Gefäße vor dem Füllen zuverlässig erkannt und ausgeschieden werden. Hierzu sind zahlreiche Verfahren und Vorrichtungen bekannt (DE-OS 30 36 502, DE-PS 36 21 976), die eine berührungslose, zumeist optoelektronische Erkennung von Verunreinigungen und Fremdkörpern an bzw. in leeren Gefäßen, wie z.B. Glasflaschen, ermöglichen. Ferner können auch Beschädigungen, wie z.B. Ausbrüche oder Risse, erkannt werden. Allerdings können speziell bei den in der Getränkeindustrie verwendeten Mehrwegflaschen aus Glas oder Kunststoff im Laufe der Zeit Defekte auftreten, die auf optoelektronischem Wege nicht immer zuverlässig oder nur mit sehr hohem Aufwand erkennbar sind. Derartige Defekte können feine Haarrisse oder kleine Löcher im Boden-, Seitenwand- oder Mündungsbereich von Flaschen aus durchsichtigem Material sein, die sie für eine Wiederbefüllung unbrauchbar machen, da vor allem bei kohlensäurehaltigen Getränken, die unter Gegendruck abgefüllt werden, die Dichtigkeit der gefüllten Flaschen nicht mehr gewährleistet ist, so daß nach dem Füllen der Innendruck langsam abfallen kann. Die genannten Defekte können von der Temperaturbelastung beim vorhergehenden Reinigungsvorgang oder der Druckbelastung beim Füllen entstehen und mit jedem weiteren Umlauf einer Mehrwegflasche verstärkt werden.

Um solchermaßen schadhafte Flaschen in einer Füllinie erkennen und ausschleusen zu können, wurde bereits eine Maschine (US-PS 30 10 310) zum Testen der Druckfestigkeit von Glasflaschen vorgeschlagen. Diese mit hohem Druck arbeitende Prüfmaschine ist für dünnwandige oder aus einem flexiblen Material, insbesondere Kunststoff (z.B. Polyethylenterephthalat), bestehende Flaschen ungeeignet. Ferner ist keine Erkennung von äußerlichen, die Dichtigkeit von Flaschen nicht beeinflussenden, Beschädigungen möglich. Eine Prüfeinrichtung zur Erkennung von Schmutz, Fremdkörpern, Reinigungslauge oder Restflüssigkeit in leeren Flaschen ist nicht vorhanden.

Eine Inspektionsmaschine für Kunstofftaschen, die zusätzlich zur Dichteüberprüfung, von außen an der Flasche erkennbare Parameter (Flaschenhöhe, Rechtwinkeligkeit des Flaschenbodens, Flaschencode, Rechtwinkeligkeit von Hals und öffnung) überprüft ist in EP-A-0295371 beschieben.

Der Erfindung liegt daher die Aufgabe zugrunde, die Erkennungssicherheit einer gattungsgemäßen Inspektionsmaschine derart weiterzubilden, daß verschmutzte oder undichte Gefäße bei hoher Leistung zuverlässig mit geringem Aufwand erkannt werden können.

Diese Aufgabe wird durch die im Kennzeichen des Anspruchs 1 angegebenen Merkmale gelöst.

In der Inspektionsmaschine wird die Dichtigkeit der Gefäße geprüft, um Fehler oder Beschädigungen, die auf optoelektronischem Wege nicht mit ausreichender Sicherheit oder nur mit sehr hohem Aufwand erfaßbar sind, aufzuspüren, während andererseits Fehler, wie z.B. Schmutzflecken, die durch eine Dichtigkeitsprüfung, nicht erkennbar sind, durch berührungslos, optoelektronisch arbeitende Inspektionseinrichtungen festgestellt werden.

Zur Überprüfung der Dichtigkeit wird eine Druckprüfung durchgeführt, wozu ein verhältnismäßig niedriger Druckauf- oder -abbau im Innenraum der zu kontrollierenden Gefäße genügt. Nachfolgend wird der Druckverlauf während einer verhältnismäßig kurzen Zeitspanne gemessen. Dadurch ist ein evtl. unzulässig hoher, auf einen Gefäßfehler hinweisender Druckabfall erfaßbar. Der Prüfdruck kann bei einer Überdruckprüfung erheblich niedriger gewählt werden, als der zum Abfüllen von kohlensäurehaltigen Getränken notwendige Gegendruck zum Vorspannen der Gefäße. Die bei dieser Dichtigkeitsprüfung auftretende mechanische Belastung der Gefäße ist vorteilhafterweise gering und daher gefäßschonend.

Wird gemäß einer vorteilhaften Weiterbildung die Dichtigkeitsprüfung und die optoelektronische Prüfung teilweise oder ganz zeitgleich durchgeführt, ist eine besonders kompakte, platz- und kostensparende Bauweise möglich.

Die Dichtigkeitsprüfung kann besonders dann mit äußerst minimalem Aufwand durchgeführt werden, wenn eine axiale Einspannung zwischen Boden und Mündung der zu prüfenden Gefäße vorgesehen wird, da dann zeitgleich während der Dichtigkeitsprüfung ohne zusätzlichen Aufwand eine Seitenwandkontrolle, insbesondere nach dem Durchlichtverfahren, durchgeführt werden kann, ohne daß es zu einer negativen gegenseitigen Beeinflussung der beiden Prüfvorgänge kommt.

Die Dichtigkeitsprüfung kann aber auch während anderen, ebenfalls berührungslos, optoelektronisch durchgeführter Prüfvorgängen, wie z.B. einer Bodeninspektion oder Restflüssigkeitserkennung, durchgeführt werden. Dies ist insbesondere bei einfacheren Inspektionsmaschinen ohne eine Seitenwandkontrolleinrichtung denkbar. Bei dieser Kombination kann zur Durchführung der Dichtigkeitsprüfung, d.h. Druckprüfung, auf die Gefäßmündung eine Abdichtscheibe angedrückt werden, die seitlich einen Anschluß zur Druckgaszuführung besitzt, und in axialer Richtung, d.h. in Richtung der Gefäßlängsachse, durchsichtig, z.B. durch einen Glaseinsatz, ausgeführt ist. Dadurch kann während der Dichtigkeitsprüfung das Gefäß gleichzeitig für eine Bodenkontrolle oder Restflüssigkeitserkennung axial durchleuchtet werden. Während dieses Vorganges kann das Gefäß an der Mantelfläche, vorzugsweise nahe am Hals- oder Mündungsbereich, bodenfrei gehalten werden.

Vorteile bietet eine mit Unterdruck durchgeführte Dichtigkeitsprüfung, da dann nach Abschluß der Druckmessung ein Vakuumabbau auf Atmosphärendruck im Gefäßinnenraum durch Einleiten eines Inertgases, z.B. Kohlendioxid, oder Sterilluft vorgenommen werden kann.

Nachfolgend wird ein Ausführungsbeispiel anhand der Fig. erläutert. Es zeigen:
- Fig. 1: eine schematische Draufsicht auf eine Inspektionsmaschine,
- Fig. 2: einen vertikalen Schnitt durch einen Teil der Inspektionsmaschine entlang der Linie II-II in Fig. 1,
- Fig. 3: einen vergrößerten Teilschnitt durch die Steuereinrichtung eines in Fig. 2 nur schematisch angedeuteten Zentrierkopfes und
- Fig. 4 bis 7: Druckverlaufdiagramme von Dichtigkeitsprüfvorgängen bei verschiedenen Defekten.

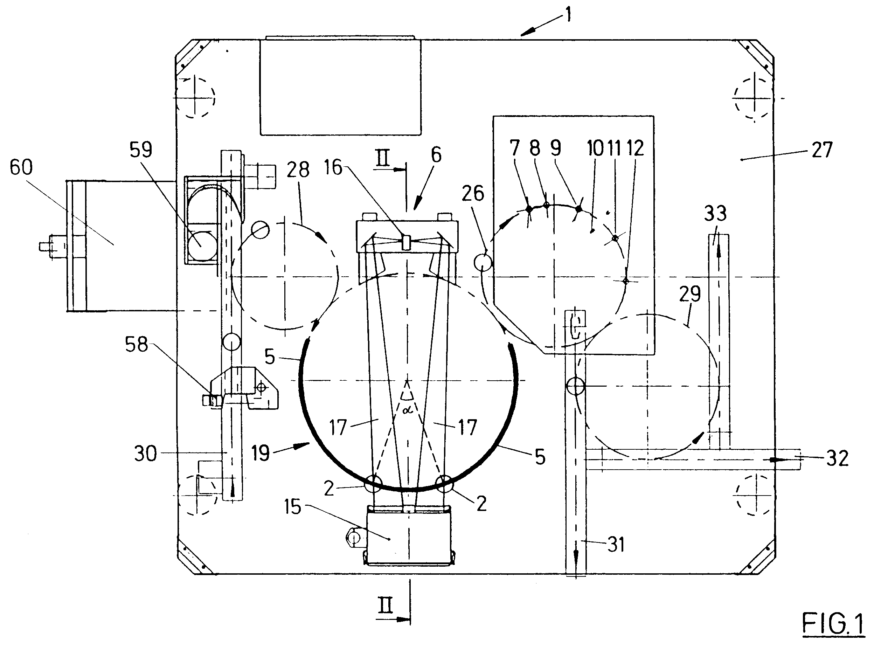

Die in Fig. 1 schematisch dargestellte Inspektionsmaschine trägt auf ihrer Tischplatte 27 ein erstes Karussell 19, auf dem die Dichtigkeitsprüfung und die Seitenwandkontrolle der Flaschen erfolgt. An das erste Karussell 19 schließt sich ein zweites Karussell 26 an, wobei sich die Teilkreise der beiden Karussells berühren. An der Umlaufbahn des zweiten Karussells 26 sind weitere Inspektionsstationen stationär angeordnet. In Flaschenlaufrichtung gesehen sind dies eine Bodenkontrolle 7, eine Infrarotkontrolleinrichtung 8 zur Restflüssigkeitserkennung, eine Decodierinspektion 9 zur Erkennung eines am Bodenbereich einer Flasche angebrachten Codes, eine Hochfrequenz-Restflüssigkeitserkennung 10, eine Mündungsinspektion 11 und eine Gewindeinspektion 12. Zum Zu- und Abführen der Flaschen ist dem ersten Karussell ein Einlaufstern 28 und dem zweiten Karussell 26 ein Auslaufstern 29 zugeordnet. Sowohl der Einlaufstern 28 als auch der Auslaufstern 29 sind in bekannter Weise mit selektiv ansteuerbaren Halteorganen zum Festhalten bzw. Freigeben der Flaschen ausgestattet.

Am ersten Karussell 19 ist die aus einer Beleuchtungseinrichtung 15 und einem Sensor 16 zur Bildaufnahme bestehende Seitenwandkontrolleinrichtung 6 angeordnet. Die Beleuchtungseinrichtung 15 und der als Zeilenkamera ausgebildete Sensor 16 sind einander gegenüberliegend außerhalb am Karussell 19 positioniert, wobei das Karussell 19 im Bereich der beiden Strahlengänge 17 einbautenfrei ist. Mit dem Bezugszeichen 5 ist die Wegstrecke markiert, während der die Dichtigkeitsprüfung der Flaschen auf dem Karussell 19 erfolgt.

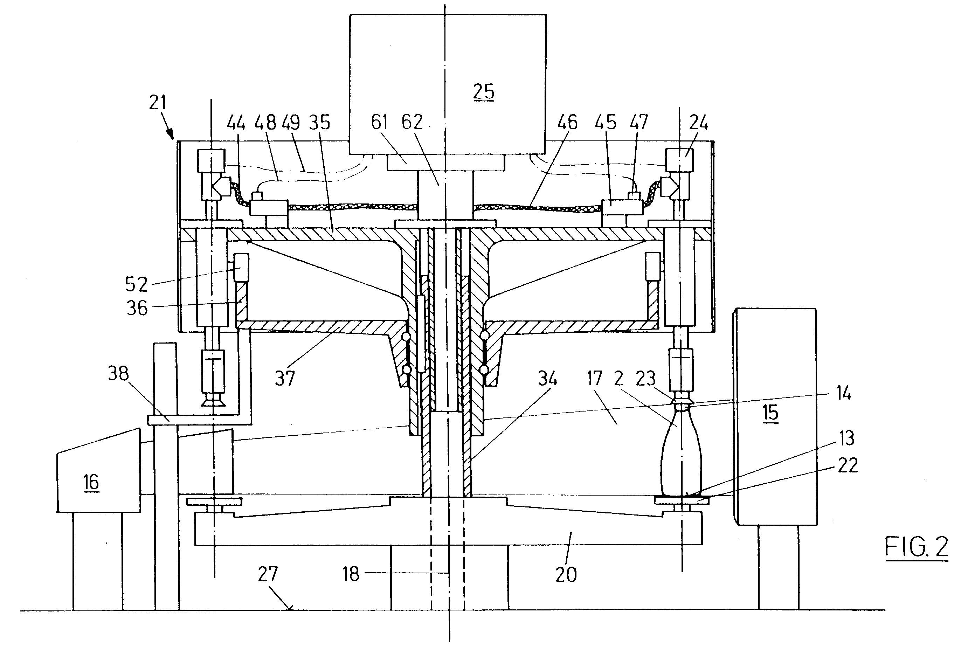

In Fig. 2 ist das erste Karussell 19 in einem Vertikalschnitt näher erkennbar. Es besteht im wesentlichen aus dem um die vertikale Achse 18 umlaufend antreibbaren Drehtisch 20 mit darauf rotierend antreibbaren Tellern 20 zur Aufnahme der Flaschen 2 und einem darüber koaxial angeordneten Rotor 21. Über die innen hohle Mittelsäule 34 ist der Rotor 21 verdrehfest mit dem Drehtisch 20 verbunden, kann aber zur Anpassung an unterschiedliche Flaschenhöhen gegenüber dem Drehtisch 20 höhenmäßig verstellt werden. Der Rotor 21 besteht im wesentlichen aus einer mit der Mittelsäule 34 verdrehfest verbundenen Mitnehmerscheibe 35, an deren äußeren Rand auf einem gemeinsamen Teilkreis mit gleichmäßigem Teilungsabstand höhenbeweglich steuerbare Zentrierköpfe 23 angeordnet sind. Die Zentrierköpfe 23 sind den Tellern 22 fluchtend zugeordnet und dienen zum axialen Einspannen der Flaschen 2. Die Höhensteuerung der Zentrierköpfe 23 erfolgt durch eine ortsfest gehaltene Hubkurve 36. Die Hubkurve 36 zum Anheben und Absenken der Zentrierköpfe 23 ist starr mit einem an der Mitnehmerscheibe 35 drehbar gelagerten Hubkurventräger 37 verbunden, der durch eine Drehmomentstütze 38 gegen Verdrehen gesichert wird. Auf der Oberseite der Mitnehmerscheibe 35 ist eine Steuer- und Auswerteinrichtung 25 zusammen mit einem Elektroverteiler 61 und einem Luftverteiler 62 befestigt. Diese Auswerteinrichtung 25 läuft zusammen mit der Mitnehmerscheibe 35 und den daran gelagerten Zentrierköpfen um. Die Strom- und Luftzufuhr zum Luftverteiler 62 bzw. Elektroverteiler 61 und der Auswerteinrichtung 25 erfolgt von unten durch die hohle Mittelsäule 34.

Außerhalb des Drehtisches 20 sind ortsfest eine Beleuchtungseinrichtung 15 und eine Zeilenkamera 16 angeordnet.

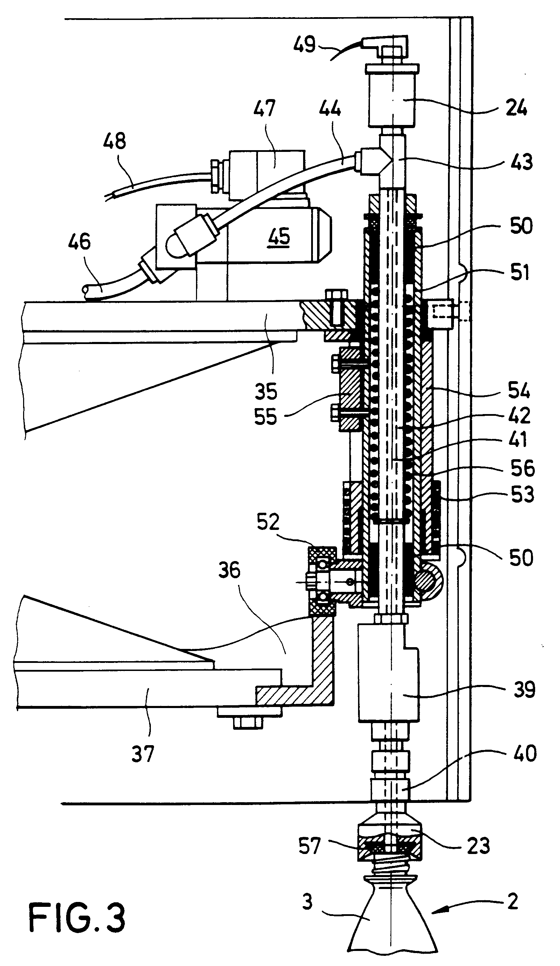

In Fig. 3 ist die Höhensteuereinrichtung der Zentrierköpfe detaillierter dargestellt. Der mit einer durchgehenden Axialbohrung 40 ausgestattete Zentrierkopf 23 ist drehbar an einem Drehverteiler 39 gelagert, dessen Gehäuse starr, d.h. verdrehfest an der ebenfalls mit einer durchgehenden Axialbohrung 41 ausgestatteten Stange 42 befestigt ist. Die Stange 42 trägt an ihrem oberen Ende ein T-Stück 43, auf dem wiederum ein Gasdrucksensor 24 befestigt ist. Vom T-Stück 43 führt eine flexible Druckluftleitung 44 zu einem auf der umlaufenden Mitnehmerscheibe 35 befestigten Druckluftventil 45, das durch eine vom Luftverteiler 62 kommende Luftversorgungsleitung 46 mit steriler Druckluft versorgt wird. Die Betätigung des Druckluftventils 45 erfolgt durch einen Steuermagneten 47. Der Steuermagnet 47 und der Gasdrucksensor 24 sind durch eine Steuerleitung 48 bzw. eine Meßleitung 49 mit der Steuer- und Auswerteinrichtung 25 verbunden.

Die Stange 42 ist verschiebbar mittels Buchsen 50 in einem Rohr 51 geführt, das an seinem unteren Ende eine Kurvenrolle 52 trägt, die, durch eine Druckfeder 53 beaufschlagt, an der Hubkurve 36 anliegt. Das Rohr 51 ist in einer mit der Mitnehmerscheibe 35 starr verbundenen Führung 54 höhenverschiebbar geführt und durch einen in einer Ausnehmung der Führung 54 gleitenden Kulissenstein 55 gegen Verdrehen gesichert. Die Stange 42 im Inneren des Rohres 51 wird permanent durch eine Druckfeder 56 nach unten in Richtung zur Flasche 2 beaufschlagt. Durch die Druckfeder 56 können unvermeidliche Höhentoleranzen der Flaschen 2 ausgeglichen werden. Zugleich ist die Federkennlinie der Druckfeder 56 so ausgelegt, daß -trotz der Toleranzunterschiede- eine im wesentlichen gleichbleibend hohe Anpressung der Zentrierdichtung 57 in den Zentrierköpfen 23 gegen die Mündung der Flaschen 2 gewährleistet wird.

Nachfolgend wird ein vollständiger Prüfzyklus einer durch die Inspektionsmaschine laufenden Flasche 2 erläutert:

Eine durch das Einlaufförderband 30 der Inspektionsmaschine 1 zugeführte Flasche 2 wird mittels einer nicht dargestellten Einteilschnecke auf die Maschinenteilung gebracht, wobei sie an einer Höhenerkennungskamera 58 vorbeigeführt wird, die vom zulässigen Höhentoleranzbereich abweichende Flaschen erkennen kann. Liegt die Flasche 2 im zulässigen Höhentoleranzbereich, wird durch die Höhenerkennungskamera 58 das zugeordnete Halteorgan, z.B. Drehriegel, am Einlaufstern 28 aktiviert und die Flasche 2 vom Einlaufförderband 30 auf den Drehtisch 20 des ersten Karussells 19 überführt. Durch die Höhenerkennungskamera 58 als fehlerhaft erkannte Flaschen werden durch den Einlaufstern 28 nicht erfaßt und gleiten durch einen in die Tischplatte 27 integrierten Fallschacht 59 in einen darunter angeordneten Sammelbehälter 60.

Bei der Übergabe einer Flasche 2 durch den Einlaufstern 28 auf den Drehtisch 20 des Karussells 19 wird die Flasche durch einen mittels der Hubkurve 36 abgesenkten Zentrierkopf 23 axial auf dem Teller 22 festgespannt. Zugleich wird durch die Steuer- und Auswerteinrichtung 25 über die Steuerleitung 48 der Steuermagnet 47 aktiviert, so daß das Druckluftventil 45 die Luftversorgungsleitung 46 mit der Druckluftleitung 44 verbindet, so daß über das T-Stück 43, die Stange 42, den Drehverteiler 39 und den Zentrierkopf 23 Sterilluft in den Innenraum 3 der Flasche 2 eingeleitet wird. Nach erfolgtem Druckaufbau im Innenraum 3 der Flasche 2 wird das Druckluftventil 45 durch den Steuermagneten 47 geschlossen, wodurch eine weitere Druckluftzufuhr unterbunden wird. Im weiteren Verlauf kann die Auswerteinrichtung 25 über die Meßleitung 49 und den daran angeschlossenen Gasdrucksensor 24 den Druckverlauf über einen gewissen Zeitraum im Innenraum 3 der Flasche 2 verfolgen. Wird dabei ein unzulässig hoher Druckabfall festgestellt, ist dies ein Hinweis auf eine defekte Flasche, die am Auslauf der Inspektionsmaschine 1 ausgeschieden werden muß.

Während die Flasche 2 zur Dichtigkeitsprüfung die Wegstrecke 5 auf dem ersten Karussell 19 zurücklegt, erfolgt gleichzeitig beim Passieren der Strahlengänge 17 die Seitenwandkontrolle im Durchlichtverfahren. Hierzu wird die Flasche 2 im Winkelbereich Alpha (ca. 35 Grad) des Drehtisches 20 vor der Beleuchtungseinrichtung 15 mit hoher Winkelgeschwindigkeit um die eigene Achse gedreht, wobei die Flasche 2 in einem schmalen Bereich entlang ihrer Mittelachse über die gesamte Höhe durchleuchtet und inspiziert wird.

Um eine vollständige Abwicklung der Flaschenmantelfläche bei der Seitenwandkontrolle zu erhalten, wird die Flasche während der Seitenwandkontrolle -je nach Art des verwendeten Sensors 16, z.B. Zeilen- oder Flächenkamera- um mindestens 180 Grad oder 360 Grad um die eigene Achse gedreht.

Nach Abschluß der Dichtigkeitsprüfung und der Seitenwandkontrolle wird die Flasche 2 dem zweiten Karussell 26 zur Durchführung weiterer Inspektionsvorgänge zugeführt, wobei im Berührpunkt der Teilkreise der beiden Karussells 19 und 26 die Flasche 2 durch das zweite Karussell 26 am Mantel- und Kopfbereich boden- und kopffrei eingespannt wird, während gleichzeitig der Zentrierkopf 23 des ersten Karussells 19 durch die Hubkurve 36 angehoben wird. Durch die boden- und mündungsfreie Einspannung können nachfolgend Boden-, Restflüssigkeits-, Gewinde- und Codierinspektionsvorgänge durchgeführt werden, indem die Flasche 2 an stationär angebrachten Inspektionsstationen vorbeigeführt wird. Entsprechend dem Inspektionsergebnis kann der Auslaufstern 29 die Flaschen auf verschiedene Auslaufförderbänder 31 bis 33 übergeben. Zu diesem Zweck besitzt er -wie der Einlaufstern 28- selektiv ansteuerbare Halteorgane, z.B. schwenkbare Drehriegel.

Alle genannten Prüfvorgänge erfolgen während dem kontinuierlichen Transport der Gefäße durch die Maschine.

Entsprechend dem zuvor beschriebenen Ausführungsbeispiel kann die Ansteuerung des Druckluftventils 45 durch die programmierbare Steuer- und Auswerteinrichtung 25 derart erfolgen, daß sowohl die Zeitspanne für den Druckaufbau im Innenraum einer Flasche als auch die Zeitspanne für die weitere Beobachtung des Druckverlaufes unabhängig von der momentanen Maschinenleistung konstant bleibt. Anstelle dessen kann aber auch die Ansteuerung des Druckluftventils 45 durch an der Umlaufbahn des Karussells 19 angeordnete Steuernocken auf mechanischem Wege erfolgen.

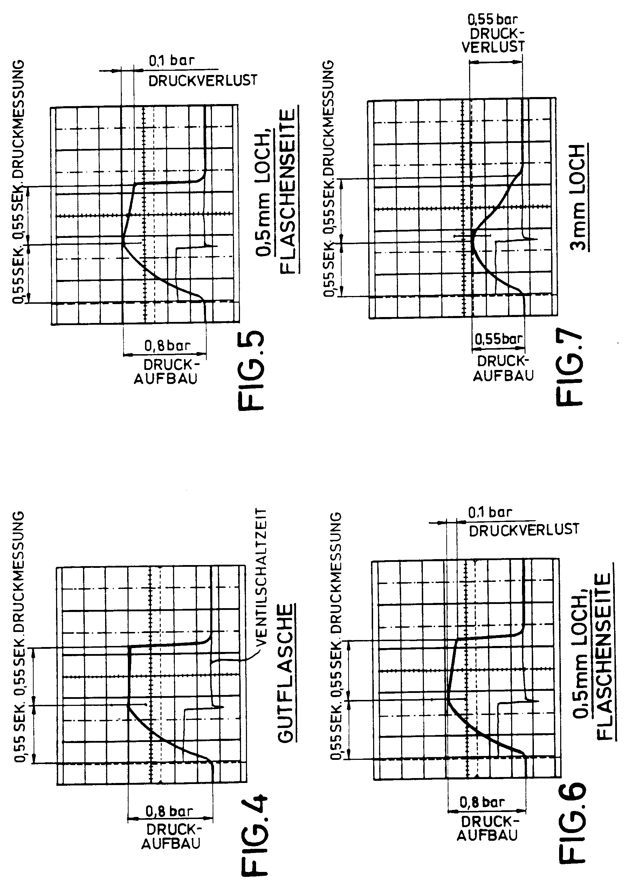

In den Fig. 4 bis 7 sind verschiedene Druckverlaufkurven am Beispiel einer 1,5 Liter PET-Flasche dargestellt. Das Diagramm in Fig. 4 zeigt den korrekten Druckverlauf einer schadlosen Flasche. In den Diagrammen nach den Fig. 5 bis 7 sind die Auswirkungen verschiedener Defekte auf den Druckverlauf erkennbar. Zur Erkennung dieser Fehler genügt beispielsweise ein Prüfdruck von 0,8 bar und eine Gesamtmeßzeit von 1,1 sek., wobei die Meßzeit jeweils zur Hälfte auf den Druckaufbau und die nachfolgende Druckverlaufsverfolgung entfällt.

Mit der beschriebenen Vorrichtung können auch Flaschen erkannt werden, deren Mündungsfläche nicht mehr senkrecht zur Flaschenmittelachse steht, da in diesem Fall sich zwischen der Zentrierglocke 23 und der Flaschenmündung infolge der gleichbleibenden Anpreßkraft ein nicht ausreichend abgedichteter Spalt ergibt, der während der Dichtigkeitsprüfung einen spürbaren Druckabfall bewirkt.

Die programmierbare Steuer- und Auswerteinrichtung 25 besitzt einen Parameterspeicher für verschiedene Flaschenformate und -sorten. Der Prüfdruck ist den Anforderungen entsprechend zentral einstellbar.

Die Dichtigkeitsprüfung kann auch durch Einsatz von Vakuum erfolgen. Dazu ist ebenfalls die vorhergehend beschriebene Inspektionsmaschine verwendbar. Als besonders günstig hat sich ein Prüfdruck im Bereich von 80 mbar (unter Atmosphärendruck) erwiesen. Günstigerweise kann nach Abschluß der Dichtigkeitsprüfung beispielsweise Kohlendioxid durch den Zentrierkopf 23 zum Druckausgleich in die Flasche geleitet werden, was einem nachfolgenden Füllvorgang zugute kommt. Die beim Befüllen von PET-Flaschen vor dem Einlassen des Füllguts übliche Gasspülung zum Verdrängen des unerwünschten Luftsauerstoffs kann wegen des bereits reduzierten Sauerstoffanteils in der Flasche in kürzerer Zeit erfolgen.

## Patentansprüche

1. Kontinuierlich arbeitende Inspektionsmaschine (1) für Gefäße (2), insbesondere für Flaschen aus transparentem Kunststoff oder Glas, mit mindestens einer stationär angeordneten optoelektronischen Inspektionseinrichtung (6 bis 12) zur Erkennung von Verunreinigungen und Beschädigungen am Boden- und/oder Seitenwand- und/oder Mündungsbereich von Gefäßen, dadurch gekennzeichnet, daß die Gefäße (2) in der Inspektionsmaschine (1) zur Erkennung von Fehlern oder Beschädigungen, die die Dichtigkeit der Gefäße beeinträchtigen, einer Dichtigkeitsprüfung (5) unterzogen werden.

2. Inspektionsmaschine nach Anspruch 1, dadurch gekennzeichnet, daß sich die Dichtigkeitsprüfung (5) und die Erkennung von Verunreinigungen oder Beschädigungen (6 bis 12) an Gefäßen (2) zumindest teilweise oder ganz überlappen.

3. Inspektionsmaschine nach Anspruch 2, dadurch gekennzeichnet, daß mindestens eine Inspektionseinrichtung (6) zur optoelektronischen Seitenwandkontrolle der Gefäße (2), insbesondere nach dem Durchlichtverfahren, vorhanden ist.

4. Inspektionsmaschine nach einem der Ansprüche 1 bis 3, dadurch gekennzeichnet, daß die Dichtigkeit der Gefäße (2) durch Beaufschlagung des Gefäßinnenraumes (3) mit einem unter Überdruck stehenden Gas oder durch Erzeugung von Unterdruck im Gefäßinnenraum (3) und gleichzeitiger Messung des Innendruckverlaufes überprüft wird.

5. Inspektionsmaschine nach Anspruch 4, dadurch gekennzeichnet, daß der Gefäßinnenraum (3) zunächst bis zum Erreichen eines einstellbaren Druckes mit Gas gefüllt oder evakuiert und zumindest anschließend, nach Beendigung der Gaszu- oder -abfuhr, der Druckabfall oder Druckanstieg während eines einstellbaren Meßintervalls erfaßt wird.

6. Inspektionsmaschine nach Anspruch 5, dadurch gekennzeichnet, daß die Gaseinfüll- oder Gasabsaugdauer und/oder das Meßintervall zur Erfassung des Innendruckverlaufes unabhängig von der momentanen Maschinenleistung zeitkonstant ist.

7. Inspektionsmaschine nach wenigstens einem der vorhergehenden Ansprüche 3 bis 6, dadurch gekennzeichnet, daß die Gefäße (2) beim Passieren einer stationären Seitenwandkontrolleinrichtung (6) mit hoher Winkelgeschwindigkeit um die eigene Achse, insbesondere mindestens um 180 Grad, gedreht werden.

8. Inspektionsmaschine nach wenigstens einem der vorhergehenden Ansprüche, dadurch gekennzeichnet, daß die Gefäße (2) zur Dichtigkeitsprüfung (5) und Seitenwandkontrolle (6) axial zwischen Boden (13) und Mündung (14) eingespannt gehalten werden.

9. Inspektionsmaschine nach wenigstens einem der Ansprüche 3 bis 8, dadurch gekennzeichnet, daß die Seitenwandkontrolleinrichtung (6) eine stationäre Beleuchtungseinrichtung (15) und mindestens einen zugeordneten Sensor (16) zur Bildaufnahme umfaßt, wobei die Inspektionsmaschine (1) höhenmäßig zwischen Mündung (14) und Boden (15) der Gefäße (2) im Strahlengangbereich (17) der Seitenwandkontrolleinrichtung (6) zwischen Beleuchtungseinrichtung (15) und Sensor (16) einbautenfrei ist.

10. Inspektionsmaschine nach wenigstens einem der vorhergehenden Ansprüche, dadurch gekennzeichnet, daß die Inspektionsmaschine (1) mindestens ein um eine vertikale Achse (18) rotierend antreibbares Karussell (19), bestehend aus einem Drehtisch (20) mit darauf drehbar befestigten Tellern (22) und einem mit Abstand koaxial darüber angeordneten Rotor (21) mit heb- und senkbaren, den Tellern (22) zugeordneten Zentrierköpfen (23), besitzt, wobei die Gefäße (2) zwischen Teller (22) und Zentrierkopf (23) axial einspannbar und durch den Zentrierkopf (23) druckbeaufschlagbar sind.

11. Inspektionsmaschine nach Anspruch 10, dadurch gekennzeichnet, daß jedem Zentrierkopf (23) ein Gasdrucksensor (24) zugeordnet ist, der mit einer auf dem Rotor (21) mitumlaufenden Auswerteinrichtung (25) in Verbindung steht.

12. Inspektionsmaschine nach Anspruch 11, dadurch gekennzeichnet, daß die stationäre Seitenwandkontrolleinrichtung (6) eine radial außerhalb am Drehtisch (20) angeordnete Beleuchtungseinrichtung (15) und mindestens einen dieser gegenüberliegenden Sensor (16) zur Bildaufnahme, der vorzugsweise ebenfalls radial außerhalb am Drehtisch angeordnet ist, umfaßt.

13. Inspektionsmaschine nach wenigstens einem der vorhergehenden Ansprüche, dadurch gekennzeichnet, daß die Inspektionsmaschine (1) wenigstens ein die Gefäße (2) bodenfrei tragendes, um eine vertikale Achse rotierend antreibbares Karussell (26) aufweist, wobei an der Umlaufbahn des Karussells (26) Inspektionseinrichtungen (7 bis 12) zur Boden- und/oder Mündungs- und/oder Gewindekontrolle und/oder Restflüssigkeitserkennung stationär angeordnet sind.

14. Inspektionsmaschine nach Anspruch 13, dadurch gekennzeichnet, daß die Gefäße (2) im Karussell (26) an der Mantelfläche, vorzugsweise nahe am Kopf- oder Mündungsbereich, gehalten werden.

15. Inspektionsmaschine nach Anspruch 10 bis 14, dadurch gekennzeichnet, daß die beiden Karussells (19, 26) nebeneinander angeordnet sind und von den Gefäßen (2) nacheinander durchlaufen werden, wobei die Gefäße vorzugsweise unmittelbar von einem Karussell zum anderen übergeben werden.

16. Inspektionsmaschine nach einem der vorhergehenden Ansprüche, dadurch gekennzeichnet, daß die Dichtigkeitsprüfung (5) ganz oder teilweise während einer Bodenkontrolle (7) und/oder Restflüssigkeitserkennung (8, 10) erfolgt.

17. Inspektionsmaschine nach wenigstens einem der vorhergehenden Ansprüche, dadurch gekennzeichnet, daß nach Abschluß einer Dichtigkeitsprüfung mittels Unterdruck der Gefäßinnenraum (3) durch Einleiten von Sterilluft oder eines Inertgases, vorzugsweise Kohlendioxid, auf Atmosphärendruck entlastet wird.

## Claims

1. Continuously operating inspection machine (1) for containers (2), especially for bottles made of transparent plastic or glass, having at least one optoelectronic inspection device (6 to 12) arranged in stationary manner for identifying impurities and instances of damage to the base and/or side-wall and/or mouth region of containers, characterized in that the containers (2) are subjected to an impermeability test (5) in the inspection machine (1) for identifying faults or instances of damage which impair the impermeability of the containers.

2. Inspection machine according to Claim 1, characterised in that the impermeability test (5) and the identification of impurities or instances of damage (6 to 12) on bottles (2) overlap completely or at least in part.

3. Inspection machine according to Claim 2, characterised in that at least one inspection device (6) is available for the optoelectronic side-wall inspection of the containers (2), especially according to the transmitted light method.

4. Inspection machine according to one of the Claims 1 to 3, characterised in that the impermeability of the containers (2) is checked by pressurising the interior (3) of the container with a gas under excess pressure or by producing a partial vacuum in the interior (3) of the container and simultaneously measuring the variation in the internal pressure.

5. Inspection machine according to Claim 4, characterised in that the interior (3) of the container is firstly filled with gas to achieve an adjustable pressure or evacuated and at least subsequently, after termination of the supply or removal of gas, the drop in pressure or rise in pressure is detected during an adjustable measuring interval.

6. Inspection machine according to Claim 5, characterised in that the duration of gas filling or gas evacuation and/or the measuring interval for detecting the internal pressure variation is a constant period independent of the instantaneous machine output.

7. Inspection machine according to at least one of the preceding Claims 3 to 6, characterised in that on passing a stationary side-wall inspection device (6) the containers (2) are rotated at high angular velocity about their own axis, especially by at least 180 degrees.

8. Inspection machine according to at least one of the preceding claims, characterised in that for the impermeability test (5) and side-wall inspection the containers (2) are held clamped axially between the base (13) and mouth (14).

9. Inspection machine according to at least one of the preceding Claims 3 to 8, characterised in that the side-wall inspection device (6) comprises a stationary illumination device (15) and at least one associated sensor (16) for image recording, the inspection machine (1) being free of installed elements in terms of vertical elevation between the mouth (14) and base (15) of the containers (2) in the region of the beam path (17) of the side-wall inspection device (6) between the illumination device (15) and sensor (16).

10. Inspection machine according to at least one of the preceding claims, characterised in that the inspection machine (1) possesses at least one carousel (19) capable of being driven in rotating manner about a vertical axis (18) and consisting of a turntable (20) having plates (22) fastened rotatably on it and a rotor (21) arranged coaxially at a distance above it having centring heads (23) capable of being raised and lowered associated with the plates (22), the containers (2) being capable of being clamped axially between the plate (22) and centring head (23) and capable of being acted on by pressure by means of the centring head (23).

11. Inspection machine according to Claim 10, characterised in that associated with each centring head (23) is a gas pressure sensor (24) which is connected to an evaluating device (25) co-rotating on the rotor (21).

12. Inspection machine according to Claim 11, characterised in that the stationary side-wall inspection device (6) comprises an illumination device (15) arranged radially on the outside of the turntable (20) and at least one sensor (16) for image recording located opposite the latter which is preferably likewise arranged radially on the outside of the turntable.

13. Inspection machine according to at least one of the preceding claims, characterised in that the inspection machine (1) comprises at least one carousel (26) carrying the containers (2) with their bases free capable of being driven in rotating manner about a vertical axis, inspection devices (7 to 12) for inspection of the base and/or the mouth and/or the screw thread and/or for detecting residual liquid being arranged in stationary manner on the circular path of the carousel (26).

14. Inspection machine according to Claim 13, characterised in that in the carousel (26) the containers (2) are held on the side area, preferably close to the head or mouth region.

15. Inspection machine according to Claim 10 to 14, characterised in that the two carousels (19, 26) are arranged beside one another and are traversed one after the other by the containers (2), the containers being transferred preferably directly from one carousel to the other.

16. Inspection machine according to one of the preceding claims, characterised in that the impermeability test (5) takes place wholly or partly during a base inspection (7) and/or detection of residual liquid (8, 10).

17. Inspection machine according to at least one of the preceding claims, characterised in that after conclusion of an impermeability test by means of partial vacuum, the interior (3) of the container is released to atmospheric pressure by introducing sterile air or an inert gas, preferably carbon dioxide

## Revendications

1. Machine d'inspection (1) en continu de récipients (2), en particulier de bouteilles en matière plastique transparente ou en verre, comprenant au moins un dispositif d'inspection optoélectronique (6 à 12) installé de manière stationnaire et destiné à détecter des impuretés et endommagements dans la région du fond et/ou de la paroi lacérale et/ou de l'embouchure de récipients, **caractérisée en** ce que les récipients (2) sont soumis dans la machine d'inspection (1) à un contrôle de l'étanchéité (5) pour détecter des défauts ou des endommagements qui compromettent l'étanchéité des récipients.

2. Machine d'inspection selon la revendication 1, caractérisée en ce que le contrôle de l'étanchéité (5) et la détection d'impuretés ou d'endommagements (6 à 12) sur des récipients (2) se recouvrent au moins en partie ou entièrement.

3. Machine d'inspection selon la revendication 2, caractérisée en ce qu'elle comprend au moins un dispositif d'inspection (6) pour le contrôle optoélectronique de la paroi latérale des récipients (2), en particulier d'après le procédé de l'éclairage en lumière transmise.

4. Machine d'inspection selon l'une des revendications 1 à 3, caractérisée en ce que l'étanchéité des récipients (2) est contrôlée par l'alimentation de l'espace intérieur (3) du récipient avec un gaz sous surpression ou par la réalisation d'une dépression dans l'espace intérieur (3) du récipient et par la mesure simultanée de la variation de la pression intérieure.

5. Machine d'inspection selon la revendication 4, caractérisée en ce que l'intérieur (3) du récipient est tout d'abord rempli de gaz ou évacué jusqu'à l'atteinte d'une pression réglable et qu'au moins ensuite, après la fin de l'amenée ou de l'évacuation de gaz, la chute de pression ou l'augmentation de la pression est mesurée pendant un intervalle de mesure réglable.

6. Machine d'inspection selon la revendication 5, caractérisée en ce que la durée d'injection ou d'aspiration de gaz et/ou l'intervalle de mesure pour l'enregistrement de la variation de la pression intérieure est constante dans le temps.

7. Machine d'inspection selon au moins l'une des revendications précédentes 3 à 6, caractérisée en ce que, lors du passage dans un dispositif de contrôle de paroi latérale (6) stationnaire, les récipients (2) sont tournés à grande vitesse angulaire autour de leur propre axe, notamment d'au moins 180 degrés.

8. Machine d'inspection selon au moins l'une des revendications précédentes, caractérisée en ce que pour le contrôle de l'étanchéité (5) et le contrôle de la paroi latérale (6), les récipients (2) sont fixés axialement entre le fond (13) et l'embouchure (14).

9. Machine d'inspection selon au moins l'une des revendications 3 à 8, caractérisée en ce que le dispositif de contrôle de paroi latérale (6) comprend un dispositif d'éclairage (15) stationnaire et au moins un détecteur (16) associé pour la prise de vues, la machine d'inspection (1) étant dépourvue d'éléments encastrés se situant en hauteur entre l'embouchure (14) et le fond (15) des récipients (2), dans la zone des marches des rayons (17) du dispositif de contrôle de paroi latérale (6) entre le dispositif d'éclairage (15) et le détecteur (16).

10. Machine d'inspection selon au moins l'une des revendications précédentes, caractérisée en ce que la machine d'inspection (1) comprend au moins un carrousel (19) pouvant être entraîné en rotation autour d'un axe vertical (18), qui se compose d'une platine tournante (20) sur laquelle sont montés de manière tournante des plateaux (22) et d'un rotor (21) disposé coaxialement au-dessus de ces derniers et comprenant des têtes de centrage (23) ascendantes et descendantes associées auxdits plateaux (22), les récipients (2) pouvant être fixés axialement entre le plateau (22) et la tête de centrage (23) et mis sous pression par la tête de centrage (23).

11. Machine d'inspection selon la revendication 10, caractérisée en ce qu'à chaque tête de centrage (23) est associé un détecteur de pression de gaz (24) qui est relié à un dispositif d'interprétation (25) solidaire en rotation avec le rotor (21).

12. Machine d'inspection selon la revendication 11, caractérisée en ce que le dispositif de contrôle de paroi latérale (6) stationnaire comprend un dispositif d'éclairage (15) installé radialement à l'extérieur sur la platine tournante (20), et au moins un détecteur (16) pour la prise de vues placé en face du dispositif d'éclairage et monté de préférence également radialement à l'extérieur sur la platine tournante.

13. Machine d'inspection selon au moins l'une des revendications précédentes, caractérisée en ce que la machine d'inspection (1) comprend au moins un carrousel (26) portant les récipients (2) avec le fond libre et pouvant être entraîné en rotation autour d'un axe vertical, des dispositifs d'inspection (7 à 12) pour le contrôle du fond et/ou de l'embouchure et/ou pour la détection de liquide résiduel étant installés de manière stationnaire sur la périphérie du carrousel (26).

14. Machine d'inspection selon la revendication 13, caractérisée en ce que les récipients (2) sont maintenus dans le carrousel (26) par la surface latérale, de préférence à proximité de la zone de tête ou de l'embouchure.

15. Machine d'inspection selon les revendications 10 à 14, caractérisée en ce que les deux carrousels (19, 26) sont disposés l'un à côté de l'autre et qu'ils sont parcourus successivement par les récipients (2), lesdits récipients étant de préférence transférés directement d'un carrousel à l'autre.

16. Machine d'inspection selon l'une des revendications précédentes, caractérisée en ce que le contrôle de l'étanchéité (5) est effectué entièrement ou en partie pendant un contrôle de fond (7) et/ou une détection de liquide résiduel (8, 10).

17. Machine d'inspection selon au moins l'une des revendications précédentes, caractérisée en ce que, après la fin d'un contrôle de l'étanchéité par dépression, l'espace intérieur (3) du récipient est ramené à la pression atmosphérique par l'injection d'air stérilisé ou d'un gaz inerte, de préférence de dioxyde de carbone.
